# EUROPÄISCHE PATENTSCHRIFT

(11) **EP 0 331 786 B1**
(45) Veröffentlichungstag und Bekanntmachung des Hinweises auf die Patenterteilung: **03.08.1994**
(21) Anmeldenummer: 88103884.8
(22) Anmeldetag: 11.03.1988
(51) Int. Cl.: C08H 1/06, A61L 15/16, A61L 27/00, A61L 25/00

(54) **Verfahren zur Herstellung von Kollagenmembranen für Hämostase, Wundbehandlung und Implantate**
Method for the preparation of collagen membranes for haemostasis, wound treatment and implants
Procédé pour la préparation de membranes de collagène pour hémostase, traitement de blessures, et implants

(43) Veröffentlichungstag der Anmeldung: 13.09.1989
(73) Patentinhaber: CHEMOKOL G.b.R. Ing.-Büro für Kollagenprodukte, D-52222 Stolberg (DE)
(72) Erfinder: Eckmayer, Zdenek, D-6940 Weinheim (DE); Janzen, Ernst, 1251 GR Laren (NL); Rüttgers, Günter, D-5190 Stolberg (DE)
(74) Vertreter: Brauns, Hans-Adolf, Dr. rer. nat.

(56) Entgegenhaltungen:
- AU-B- 77 566
- DE-A- 2 730 623
- DE-A- 3 203 957

## Beschreibung

Die Erfindung betrifft die Herstellung von medizinschen Membranen, die für die Wundbehandlung, Blutstillung oder als Implantationsmaterial geeignet sind.

Kollagen wird schon seit langem für medizinische und insbesondere chirurgische Zwecke angewendet. Das Kollagen liegt dabei entweder als Pulver vor (US-PS 3 742 955 und 3 810 473) oder in Form eines Schwammes (US-PS 4 320 201 und DE-PS 2 625 289) und kann schließlich auch in Form eines Vlieses (DE-PS 27 30 623) vorliegen. Die Pulver, Schwämme und Vliese aus Kollagen haben in der Medizin Bedeutung erlangt, weil Kollagen als körpereigener Stoff körperfremden Stoffen vorzuziehen ist. Kollagenschwämme weisen eine gute Saugfähigkeit auf und ein niedriges spezifisches Gewicht und passen sich gut einer Wunde an. Die Anhaftung an der Wunde ist oft unbefriedigend. Außerdem sind Schwämme zwar zur Blutstillung bei einer Sickerblutung geeignet, nicht aber, um bei einer Blutung, bei welcher das Blut unter Druck ausströmt, eingesetzt zu werden.

Aus der EP-PS 0 070 398 ist ein Verfahren zur Herstellung von Kollagenmaterial für chirurgische Zwecke bekannt, bei dem Achillessehnen oder Häute alkalisch aufgeschlossen und nach einer Säurebehandlung mechanisch behandelt, entquollen, vernetzt und getrocknet werden. Aus einem solchen Kollagenmaterial werden die einzelnen längsorientierten Fasern freigelegt, die dann auf Textilmaschinen in für Textilien bekannter Weise weiterverarbeitet werden können. Auf diese Weise kann man ein chirurgisches Material für zahlreiche Anwendungsmöglichkeiten erhalten. Da das spezifische Gewicht eines solchen Produktes wesentlich größer ist als bei einem Schwamm, kann man mit diesem Material auch eine schnellere Hämostase erzielen als mit einem Kollagenschwamm. Die mechanische Festigkeit bei schwierigen Blutungen, bei denen das Blut unter Druck ausströmt (vasculare Anastomosen), ist jedoch immer noch nicht ausreichend, insbesondere auch, wenn man verletzte innere Organe in einem hämostatischen Netz zusammenhalten will. Auch die Biegsamkeit des Materials läßt oft zu wünschen übrig, so daß die Anpassungsfähigkeit an die Wunde nicht immer befriedigt.

Aufgabe der vorliegenden Erfindung ist es, medizinische Membrane zur Verfügung zu stellen, die aus feinen Fasern aufgebaut sind, bei denen aber der native, integrale Zusammenhang erhalten geblieben ist und die eine schnelle hämostatische Wirkung ergeben, die schmiegsam sind und sich ausgezeichnet einer Wunde anpassen, die eine schnell quellbare Oberfläche haben, die außerdem geschmeidig sind und die gut an der blutenden Oberfläche einer Wunde anhaften. Verbunden mit dieser Aufgabe ist es, daß die Membran eine hohe mechanische Festigkeit aufweist und auf diese Weise als hämostatische Umhüllung eingesetzt werden kann, die die Form, den Zustand und den Schutz der verletzten Organe sichert. Die große mechanische Festigkeit ist insbesondere von Bedeutung, wenn die Membranen bei Blutungen, bei denen das Blut unter Druck ausströmt, zum Abdecken und Einstellen der Blutung eingesetzt werden.

Diese Aufgabe wird durch eine Kollagenmembran, die nach dem Verfahren gemäß dem Patentanspruch 1 hergestellt wurde, gelöst.

Die Erfindung betrifft somit ein Verfahren zur Herstellung von Kollagenmaterial für medizinische Zwecke, bei dem man die Kollagenrohstoffe aus Rinder- oder Schweinekollagen mechanisch von Fettresten befreit, mit verdünnten Alkalien und Säuren und gegebenenfalls Enzymen behandelt, zwischen diesen Schritten gründlich wäscht und dabei den Kollagenrohstoff von begleitenden Verunreinigungen reinigt und unter Erhalt einer Kollagenmatrix mit einem Trockengewicht von etwa 25 Gew.% weiterverarbeitet, das dadurch gekennzeichnet ist, daß man
a) die Kollagenmasse mit starkem Alkali behandelt, bis der Amidstickstoff 0,35 mmol/g oder weniger beträgt,
b) anschließend mit starker Säure bei einem pH-Wert von 1 oder weniger behandelt und dann mit Wasser wäscht unter Erhalt eines Trockengewichtes von 10 bis 14 Gew.%, wobei der pH-Wert auf 2,5 bis 3,5 ansteigt,
c) durch Zusatz einer anorganischen Salzlösung entquellt,
d) das in Stufe c) enthaltene Material bis zu einem Trockengewicht von 40 bis 50 Gew.% abquetscht,
e) die Stufen c) und d) mehrfach wiederholt,
f) anschließend die erhaltene Matrix durch Zugabe von Lösungsmittel entwässert,
g) gegebenenfalls eine Behandlung mit weiteren Stoffen (Vernetzungsmittel, Weichmacher) durchführt, und
h) schließlich die erhaltene Matrix in gespannter Form endtrocknet.

Als Rohstoff dient tierisches Kollagen, z.B. Amnion, Chorion, Omentum, Pericardium und dergleichen. Diese Produkte werden chemisch aufgeschlossen, aber es wird dafür Sorge getragen, daß die ursprüngliche Struktur der Kollagenfasern (die Matrix) erhalten bleibt. Diese Ausgangsmaterialien werden von Rindern oder Schweinen gewonnen.

Die Gewinnung von Kollagenmaterialien durch Behandeln der Ausgangsprodukte mit Alkalien, Säuren, Enzymen, Salzen, Vernetzungsmitteln und organischen Lösungsmitteln ist bekannt.

Man erhält auf diese Weise Kollagenfasern, die mehr oder weniger denaturiert sind. So wird in der DE-PS 27 30 623 die Herstellung von Kollagenfasern beschrieben, bei der das gewaschene und zerkleinerte Ausgangsmaterial zunächst alkalisch und dann sauer behandelt und dann mechanisch aufgeschlossen wird. Bei dieser Verfahrensweise erhält man jedoch ein Kollagenmaterial mit einer geringen mechanischen Festigkeit, bei dem die ursprüngliche Kollagenmatrix und damit der ursprünglich integrale Zusammenhang der Fasern vernichtet wurde. Es hat sich aber herausgestellt, daß die Eigenschaften von Kollagenmaterialien nicht nur weitgehend von der Nativität der Faser, sondern auch von der Nativität der Struktur abhängig sind. Direkt verbunden mit der Nativität der Struktur ist z.B. die Stimulierung von der Epithelisation bei der Wundbehandlung und die Unterstützung der Stimulation von dem Granulationsgewebe. Deshalb ist es bei der vorliegenden Erfindung wesentlich, daß ein hochwertiges Kollagenmaterial erhalten wird, bei dem die Struktur der Kollagenmatrix dem ursprünglichen nativen Zustand weitgehend entspricht.

Das erfindungsgemäße Verfahren besteht aus einer Reihe von Schritten, die miteinander technologisch verbunden sind. Dabei sind einige dieser Verfahrensschritte bei Verfahren zur Herstellung von Kollagenmaterialien an sich bekannt. Die genaue Art dieser Verfahrensschritte und deren Reihenfolge ist aber zur Erzielung der durch die vorliegende Erfindung angestrebten Kollagenmaterialien von Bedeutung.

Die im Oberbegriff des Patentanspruchs 1 genannten Verfahrensschritte sind allgemein bekannt. Es handelt sich hier um die Reinigung des Materials. Dieser Arbeitsschritt ist z.B. aus der Lederindustrie und der Gelatineherstellung bekannt und wird in ähnlicher Weise auch bei der Herstellung von Wursthüllen, bei der Herstellung von Kollagen-Verpackungsmaterialien und der Herstellung von Kollagenfasern angewendet. Dieses Reinigungsverfahren beinhaltet die folgenden Maßnahmen:

Zunächst wird der Kollagenrohstoff aus Rinder- oder Schweinekollagen mechanisch von Fettresten befreit. Dann wäscht man das so weitgehend vom Fett befreite Kollagenmaterial gründlich mit Wasser, vorzugsweise mit fließendem Wasser. Anschließend erfolgt eine Behandlung des Materials mit Alkali, z.B. mit einer verdünnten, etwa 0,5%-igen NaOH-Lösung. Daran kann sich dann wieder ein Waschvorgang, vorzugsweise mit fließendem Wasser, anschließen. In der nächsten Reinigungsstufe wird das Material mit einer Säure behandelt, vorzugsweise mit Salzsäure, wobei man den pH-Wert des angesäuerten Materials auf etwa 1,0 oder weniger einstellt. Dann wird wieder mit Wasser gewaschen. Man erhält auf diese Weise eine Kollagenmatrix mit einem Trockengewicht von etwa 25 Gew.%.

Die erfindungsgemäße Weiterverarbeitung sieht nun folgendes vor:
a) Die in der zuvor angegebenen Weise gereinigte Kollagenmasse wird mit starkem Alkali behandelt. Vorzugsweise verwendet man hierzu Natriumhydroxid. Diese alkalische Behandlung ist bei der Herstellung von Kollagenmaterialien an sich bekannt. Die Behandlung erfolgt zur Lockerung der Fasern, die teilweise geöffnet werden und auch teilweise chemisch modifiziert werden. Diese Alkalibehandlung erfolgt, bis der Amidstickstoff etwa auf die Hälfte der ursprünglichen Größe sinkt. Dies ist der Fall, wenn der Amidstickstoff 0,35 mmol/g Kollagen oder weniger beträgt. Diese Alkalibehandlung, die vorzugsweise in einem Gerberfaß durchgeführt wird, dauert etwa 10 bis 15 Tage.

In der Stufe b) erfolgt dann die Behandlung mit einer starken Säure. Hierbei werden die Membranen zunächst mit einer Säure, vorzugsweise Salzsäure, angesäuert. Diese Säurebehandlung dauert so lange, bis das Material homogen durchgesäuert ist, im allgemeinen etwa 1 bis 5 Stunden und vorzugsweise etwa 2 bis 3 Stunden. Man wendet dabei verhältnismäßig starke Säuren, vorzugsweise Salzsäure, in einer Konzentration von etwa 3% an. Dabei erfolgt nur eine verhältnismäßig schwache Anquellung des Materials, das nach der Säurebehandlung einen pH-Wert von unter 1,0 hat. Im Anschluß daran wäscht man wieder mit fließendem Wasser. Dabei steigt der pH-Wert des Materials auf etwa 3,0 (HCl) an und das Material quillt an. Es hat jetzt ein Trockengewicht von 10 bis 14 Gew.%.

In der Stufe c) erfolgt die Entquellung des Materials. Zu diesem Zweck werden die Membranen mit einer Lösung eines anorganischen Salzes, vorzugsweise mit Kochsalz, behandelt. Der pH-Wert dieser Lösung beträgt 2,5 bis 3,5 und vorzugsweise 3,0, und die Konzentration des Salzes beträgt 10 bis 20 Gew.%. Diese Behandlung dauert einige Minuten.

In der Stufe d) wird das in der Stufe c) erhaltene Material auf ein Trockengewicht von 40 bis 50 Gew.% abgequetscht. Dieser Vorgang hat beim erfindungsgemäßen Verfahren folgende wichtige Bedeutung: Die manchmal feste fasrige Struktur der Membranen wird durch die starke Quellung und Entquellung gelockert. Die entquollenen Kollagenfasern quellen aber sofort wieder auf, wenn man das Salz aus den Fasern auswäscht. Deshalb wird nach der Stufe d) das Salz vorzugsweise ausgewaschen.

Die Stufen c) und d) werden mehrmals wiederholt, d.h. wenigstens zwei-, aber vorzugsweise dreimal, wobei das Material dann zwei- bzw. dreimal von dem angequollenen zu dem entquollenen Zustand übergeht.

In der Stufe f) wird die erhaltene Matrix durch Zugabe eines Lösungsmittels entwässert. Es ist wichtig, daß die Trocknung der entquollenen Membranen nicht an der Luft erfolgt. Dabei würden die einzelnen Fasern und Fibrillen völlig miteinander verkleben. Derart getrocknete Membranen sind unregelmäßig transparent, haben nur eine stark verminderte Porosität und die innere Oberfläche der Membran ist wesentlich kleiner. Dies würde bedeuten, daß die Rehydratation des Materials bei der Einwirkung von hydrophilen Flüssigkeiten Stunden bedeutet, und darum sind solche Materialien in der Chirurgie nicht brauchbar. Beim erfindungsgemäßen Verfahren wird vorzugsweise die Trocknung mit Aceton durchgeführt. Durch diese Acetontrocknung wird das Wasser und die Säure vollständig abgezogen. Das Salz, soweit es nicht vorher ausgewaschen wurde, ist in Aceton nicht löslich und bleibt in den Membranen.

Die so getrocknete (entwässerte) Matrix wird anschließend in gespannter Form endgetrocknet, z.B. an der Luft.

In der Stufe g) erfolgt nun gegebenenfalls eine Behandlung mit weiteren Stoffen, z.B. mit Vernetzungsmitteln oder Weichmachern. Diese weitere Behandlung ist insbesondere von Interesse, um die erfindungsgemäßen Kollagenmaterialien für spezielle medizinische Einsatzgebiete zu modifizieren. Werden die Membrane beispielsweise für Implantate verwendet, dann ist eine Vernetzung von Interesse. Solche Vernetzungen bei Kollagenmaterialien sind an sich bekannt. Geeignete physiologisch unbedenkliche Vernetzungsmittel sind insbesondere Hexamethylendiisocyanat und Polyethylenglykoldiglycidylether. Der Vernetzungsgrad kann durch die Menge der zugegebenen Vernetzungsmittel variiert werden. Weiterhin ist es auch möglich, Weichmacher in der Stufe g) zuzugeben, z.B. Glycerin. Man erhält auf diese Weise sehr geschmeidige Polymermaterialien.

In den nachfolgenden Beispielen werden bevorzugte Ausführungsformen beschrieben. Im Beispiel 1 wird eine hämostatische Membran beschrieben, bei welcher keinerlei Vernetzung oder Weichmachung erfolgte. Gemäß Beispiel 2 wird die Herstellung einer Membran beschrieben, die insbesondere für die Wundbehandlung geeignet ist. Schließlich wird im Beispiel 3 eine vernetzte Membran beschrieben, bei der durch Vernetzen mit Hexamethylendiisocyanat eine Verfestigung erfolgte.

### Beispiel 1: Die Herstellung von hämostatischen Membranen

Die frische Peritoneum-Membranen aus Schwein werden zunächst grob vom Fett befreit, dann mit fließendem Wasser gründlich ausgewaschen. Anschließend wird das Material 24 Stunden lang mit einer 0,5%-igen NaOH-Lösung behandelt und wieder mit fließendem Wasser ausgewaschen. Danach wird das Material mit HCl angesäuert, wobei der pH-Wert des angesäuerten Materials etwa pH 1,0 beträgt. Anschließend wird das Material mit fließendem Wasser ausgewaschen, bis der pH-Wert des Materials etwa 2,5 bis 3,5 ist.

Das Material wird jetzt abgequetscht und mit einer Entfleischmaschine aus der Lederindustrie mechanisch entfettet. Das so vorbereitete Material dient nun für die Herstellung von allen Typen Membranen nach dem erfindungsgemäßen Verfahren.

Zur alkalischen Behandlung wird das Material etwa 20 Tage lang mit 0,3 Gew.% NaOH behandelt. Der Amidstickstoff des Materials sinkt nun auf den Wert 0,36 mmol/g. Die Behandlung wird in einem Gerberfaß durchgeführt und das Material wird regelmäßig gerührt.

Die gleichmäßig angequollenen Membranen werden nun mit fließendem Wasser gewaschen und anschließend im Gerberfaß mit 3 Gew.% HCl angesäuert. Der pH-Wert des Materials sinkt sehr schnell unter pH 1,0. Die Membranen werden nun unter Bewegung vollständig durchgesäuert. Das dauert etwa 2 bis 3 Stunden. Anschließend wird das Material mit fließendem Wasser so lange gewaschen, bis der pH-Wert der Membranen 2,9 beträgt. Das Trockengewicht der Membranen liegt jetzt bei 11%. Die Membranen werden nun in eine Flotte gegeben, die aus 10 Gew.% Kochsalz und 100 Gew.% Wasser besteht, beides ist auf das Membranengewicht gerechnet. Das Material bleibt 3 Stunden in der Salzlösung und wird dann abgequetscht. Danach wird das Material mit fließendem Wasser ausgewaschen, bis es salzfrei ist. Das stark angequollene Material wird wieder mit einer Kochsalzlösung wie oben beschrieben behandelt.

Der pH-Wert der Kochsalzlösung wird auf dem Wert 3,0 gehalten. Diese Vorgänge werden dreimal wiederholt, wobei das Material dreimal von dem angequollenen zu entquollenem Zustand übergeht und dadurch gelockert wird. Nach der letzten Entquellung liegen die Membranen in gelochter Form vor.

Nun wird das Material dreimal mit Aceton behandelt, wodurch es entwässert und getrocknet wird.

Zu der letzten Aceton-Flotte wurden 5 Gew.% Wasser zugegeben (auf das Membranengewicht gerechnet). Nach der Aceton-Behandlung werden die Membranen abgequetscht. Durch das zugegebene Wasser können nun die Membranen in gestrecktem Zustand getrocknet werden. Nach dem Trocknen sind die Membranen gelockert, plan und die Fasern haben die optimale Oberfläche. Das Material ist physiologisch einwandfrei und kann nach dem Sterilisieren zur Blutstillung verwendet werden.

### Beispiel 2: Transparente Membranen für die Wundbehandlung

Der Rohstoff und die chemische Behandlung werden gemäß Beispiel I durchgeführt. Der Wert des Amidstickstoffs war ebenfalls 0,36 mmol/g und die nachfolgende Behandlung bis zur Salzbehandlung entspricht ebenfalls dem Beispiel I. Die Membranen werden dreimal mit Aceton getrocknet. Zur letzten Acetonflotte werden einige Tropfen Salzsäure zugegeben. Anschließend werden die Membranen an der Luft getrocknet. Der pH-Wert des wässrigen Extraktes der Membranen beträgt pH 2,8. Anschließend werden die Membranen zweimal mit N-Hexan behandelt. Dadurch werden die letzten Reste des Fettes beseitigt, das eine Trübheit der Membranen verursachen könnte. Nach der N-Hexan-Behandlung werden die Materialien an der Luft getrocknet und anschließend gründlich mit saurem Wasser ausgewaschen. Der pH-Wert des Wassers beträgt pH 3,5. Die Membranen werden nun angequollen und in diesem Zustand werden sie auf eine Glasplatte geschlickert (ausgestoßen). Es folgt die Trocknung des Materials an der Luft. Die Kollagenfasern kleben bei der Trocknung zusammen und das Ergebnis ist eine transparente Folie, die besonders für die Wundbehandlung verwendbar ist. Sie ermöglicht die Beobachtung der Wunde nach ihrer Abdeckung durch die Membrane. Die Membranen werden zugerichet, zweimal verpackt und sind nach dem Sterilisieren sofort verwendbar.

### Beispiel 3: Die vernetzten Membranen für die Implantate oder Wundbehandlung

Rohstoff und chemische Behandlung entsprechen den Angaben in Beispiel I. Nach der Salzbehandlung wird das Material zweimal mit Aceton gewaschen. Gleich beim ersten Waschen wird zu dem Aceton Pottasche zugegeben, dasselbe beim zweiten Waschen. Nach dem Waschen beträgt der pH-Wert des Wasserextraktes 6,8 (Bestimmung: Verhältnis Wasser : Membranen ---- 80 : 20, Schütteln 20 Minuten, nach 20 Minuten Zentrifugieren und pH-Messen). Das Material wird jetzt gründlich mit fließendem Wasser ausgewaschen. Es folgt wieder eine Trocknung mit neutralem Aceton.

Die getrockneten Membranen werden jetzt in eine Flotte gegeben, die 400 Gew.% Aceton und 2 Gew.% Hexamethylendiisocyanat enthält, alles auf das Gewicht der Membranen gerechnet. Die Membranen werden 2 Stunden gründlich geschüttelt. Nach 2 Stunden werden sie gründlich ausgewaschen in fließendem Wasser, so lange, bis die letzten Spuren des HMDIIC-Geruchs verschwunden sind, und anschließend in Aceton oder nach dem Schlickern auf eine Glasplatte getrocknet. Die Schrumpfungstemperatur des Materials beträgt 68°C.

Das Material wird nun gestanzt, zweimal verpackt, sterilisiert und ist danach sofort verwendbar.

## Patentansprüche

1. Verfahren zur Herstellung von Kollagenmembranen für medizinische Zwecke, bei dem man die Kollagenrohstoffe aus Rinder- oder Schweinekollagen mechanisch von Fettresten befreit, mit verdünnten Alkalien und Säuren und gegebenenfalls Enzymen behandelt, zwischen diesen Schritten gründlich wäscht und und dabei den Kollagenrohstoff von begleitenden Verunreinigungen reinigt und unter Erhalt einer Kollagenmatrix mit einem Trocken-Trockengewicht von etwa 25 Gew.% weiterverarbeitet, dadurch **gekennzeichnet,** daß man
a) die Kollagenrohmaterialien mit starkem Alkali behandelt, bis der Amidstickstoff 0,35 mmol/g oder weniger beträgt,
b) anschließend mit starker Säure bei einem pH-Wert von 1 oder weniger behandelt und dann mit Wasser wäscht unter Erhalt eines Trockengewichtes von 10 bis 14 Gew.%, wobei der pH-Wert auf 2,5 bis 3,5 ansteigt,
c) durch Zusatz einer anorganischen Salzlösung entquellt,
d) das in Stufe c) enthaltene Material bis zu einem Trockengewicht von 40 bis 50 Gew.% abquetscht,
e) die Stufen c) und d) mehrfach wiederholt,
f) anschließend die erhaltene Matrix durch Zugabe von Lösungsmittel entwässert,
g) gegebenenfalls eine Behandlung mit weiteren Stoffen (Vernetzungsmittel, Weichmacher) durchführt, und
h) schließlich die erhaltene Matrix in gespannter Form endtrocknet.

2. Verfahren nach Anspruch 1, dadurch **gekennzeichnet,** daß man die Alkalibehandlung in der Stufe a) mit NaOH durchführt.

3. Verfahren nach Anspruch 1 oder 2, dadurch **gekennzeichnet,** daß man die Säurebehandlung mit HCl durchführt.

4. Verfahren nach Ansprüchen 1 bis 3, dadurch **gekennzeichnet,** daß man in Stufe c) als anorganische Salzlösung eine wässrige Kochsalzlösung verwendet.

5. Verfahren nach einem der vorhergehenden Ansprüche 1 bis 4, dadurch **gekennzeichnet,** daß man in Stufe f) als Lösungsmittel ein organisches Lösungsmittel verwendet.

6. Verfahren nach Anspruch 5, dadurch **gekennzeichnet,** daß man als Lösungsmittel Aceton verwendet.

7. Verfahren nach einem der vorhergehenden Ansprüche, dadurch **gekennzeichnet,** daß man in Stufe g) eine Vernetzungsbehandlung durch Zugabe eines Vernetzungsmittels durchführt.

8. Verfahren gemäß Anspruch 7, dadurch **gekennzeichnet,** daß das Vernetzungsmittel Hexamethylendiisocyanat oder Polyethylenglykoldiglycidylether ist.

9. Verfahren gemäß Ansprüchen 1 bis 8, dadurch **gekennzeichnet,** daß man als Weichmacher Glycerin zugibt.

## Claims

1. Process for producing collagen membranes for medical purposes, in which fatty residues are removed mechanically from the collagen raw materials at bovine or pig collagen, the product is treated using dilute alkalis and acids and optionally enzymes, thorough washing is carried out between these steps and the collagen raw material is thus purified of accompanying impurities and further processed to produce a collagen matrix having a dry weight of about 25 weight %, characterised in that
a) the collagen raw materials are treated using strong alkali until the amide nitrogen is 0.35 mmole/g or less,
b) then treated using strong acid at a pH value of 1 or less and then washed using water to obtain a dry weight of 10 to 14 weight %, the pH value rising to 2.5 to 3.5,
c) depleted by adding an inorganic salt solution,
d) the material present in step c) is squeezed off to a dry weight of 40 to 50 weight %,
e) steps c) and d) are repeated several times,
f) the matrix obtained is then dehydrated by adding solvent,
g) treatment with further materials (crosslinking agents, plasticisers) is optionally carried out, and
h) finally, the matrix obtained is dried to completion in stretched form.

2. Process according to claim 1, characterised in that the alkali treatment is carried out in step a) using NaOH.

3. Process according to claim 1 or 2, characterised in that acid treatment is carried out using HCl.

4. Process according to claims 1 to 3, characterised in that an aqueous sodium chloride solution is used in step c) as the inorganic salt solution.

5. Process according to one of the preceding claims 1 to 4, characterised in that an organic solvent is used in step f) as the solvent.

6. Process according to claim 5, characterised in that acetone is used as the solvent.

7. Process according to one of the preceding claims, characterised in that crosslinking treatment is carried out in step g) by adding a crosslinking agent.

8. Process according to claim 7, characterised in that the crosslinking agent is hexamethylene diisocyanate or polyethylene glycol diglycidyl ether.

9. Process according to claims 1 to 8, characterised in that glycerin is added as plasticiser.

## Revendications

1. Procédé de préparation de membranes de collagène à des fins médicales, dans lequel les matières premières collagènes venant de collagènes d'origine bovine ou porcine, sont débarrassées mécaniquement des résidus de graisse, on traite avec des alcalis et des acides dilués et, le cas échéant, des enzymes, on opère un lavage poussé entre ces étapes et l'on épure la matière première à base de collagène des impuretés les accompagnant, et, en obtenant une matrice collagène, on retraite avec un poids à sec d'à peu près 25 % en poids, caractérisé en ce que
a) l'on traite les matières premières à collagène avec un alcali fort, jusqu'à ce que la teneur en azote des amides soit de 0,35 mmol/g ou moins,
b) puis l'on traite avec un acide fort, à une valeur du pH de 1 ou moins, en opérant ensuite un lavage, en obtenant un poids à sec de 10 à 14 % en poids, la valeur du pH ayant augmentée jusqu'à 2,5 à 3,5,
c) on dégonfle par addition d'une solution saline anorganique,
d) on extrait par écrasement le produit obtenu à l'étape c), jusqu'à obtention d'un poids à sec de 40 à 50 % en poids,
e) on répète plusieurs fois les étapes c) et d),
f) on déhydrate ensuite la matrice obtenue, par addition de solvants,
g) on effectue le cas échéant un traitement avec d'autres substances, (réticulant, plastifiant), et
h) on opère enfin un séchage final de la matrice obtenue, sous une forme tendue,

2. Procédé selon la revendication 1, caractérisé en ce que l'on effectue le traitement à l'alcali de l'étape a) avec NaOH.

3. Procédé selon la revendication 1 ou 2, caractérisé en ce que l'on effectue le traitement à l'acide avec HCl.

4. Procédé selon les revendications 1 à 3, caractérisé en ce que l'on utilise à l'étape c) comme solution saline anorganique une solution d'eau salée.

5. Procédé selon l'une des revendications 1 à 4 précédentes, caractérisé en ce que l'on utilise à l'étape f) comme solvant un solvant organique.

6. Procédé selon la revendication 5, caractérisé en ce que l'on utilise comme solvant de l'acétone.

7. Procédé selon l'une des revendications précédentes, caractérisé en ce que l'on effectue à l'étape g) un traitement de réticulation par addition d'un agent réticulant.

8. Procédé selon la revendication 7, caractérisé en ce que l'agent réticulant est un diisocyanate d'hexaméthylène ou un diglycidyléther de polyéthylène glycol.

9. Procédé selon les revendications 1 à 8, caractérisé en ce que l'on ajoute comme plastifiant de la glycérine.
